# EUROPÄISCHE PATENTSCHRIFT

(11) **EP 1 882 681 B1**
(45) Veröffentlichungstag und Bekanntmachung des Hinweises auf die Patenterteilung: **22.08.2012**
(21) Anmeldenummer: 07013870.6
(22) Anmeldetag: 16.07.2007
(51) Int. Cl.: C07C 209/36, C07C 211/46

(54) **Verfahren zur Herstellung von aromatischen Aminen**
Method for manufacturing aromatic amines
Procédé destiné à la fabrication d'amines aromatiques

(30) Priorität: 29.07.2006 DE 102006035203
(43) Veröffentlichungstag der Anmeldung: 30.01.2008
(73) Patentinhaber: Bayer MaterialScience AG, 51368 Leverkusen (DE)
(72) Erfinder: Sommer, Knut, Dr., 47798 Krefeld (DE); Schwarz, Ido, Dr., 40474 Düsseldorf (DE); Bucholz, Susanne, Dr., 50767 Köln (DE); Gehlen, Franz-Ulrich, 47839 Krefeld (DE); Lago, Andre, Minghang District 201107 Shanghai (CN); Wilke, Karl-Heinz, 47447 Moers (DE); Orzesek, Holger, Dr., 45894 Gelsenkirchen (DE); Pirkl, Hans-Georg, Dr., 51377 Leverkusen (DE); Lehner, Peter, Dr., 40878 Ratingen (DE); Schubert, Stephan, Dr., 51375 Leverkusen (DE)

(56) Entgegenhaltungen:
- EP-A- 0 696 573
- GB-A- 1 452 466

## Beschreibung

Die Erfindung betrifft ein Verfahren zur adiabaten Hydrierung von Nitroaromaten zu aromatischen Aminen in der Gasphase an ortsfesten Katalysatoren, worin der umzusetzende Nitroaromat mit Wasserstoff, Wasser, ggf. Stickstoff sowie ***im Wesentlichen in Abwesenheit von dem*** aus dem Nitroaromaten hergestellten aromatischen Amin unter Druck bei erhöhter Temperatur über den Katalysator geleitet wird.

Aromatische Amine sind wichtige Zwischenprodukte, welche preiswert und in großen Mengen zur Verfügung stehen müssen. Daher müssen z.B. für die Hydrierung von Nitrobenzol Anlagen mit sehr großen Kapazitäten gebaut werden.

Die Hydrierung von Nitroaromaten ist eine stark exotherme Reaktion. So werden beispielsweise bei 200°C bei der Hydrierung von Nitroxylol zu Xylidin ca. 488 kJ mol⁻¹ und bei der Hydrierung von Nitrobenzol zu Anilin ca. 544 kJ mol⁻¹ freigesetzt. Die Abführung und Verwendung der Reaktionswärme ist sowohl aus ökologischer als auch aus wirtschaftlicher Sicht ein wichtiger Punkt bei der Durchführung von Verfahren zur Hydrierung von Nitroaromaten.

So wird in einer etablierten Verfahrensweise der Katalysator als fluidisiertes, thermostabilisiertes Bett betrieben **(**US 3 136 818**).** Der effektiven Wärmeabfuhr dieser Verfahrensweise stehen Probleme durch uneinheitliche Verweilzeitverteilung (Nitrobenzoldurchbruch) und Katalysatorabrieb gegenüber.

Enge Verweilzeitverteilungen und geringer Katalysatorabrieb sind grundsätzlich in Reaktoren mit stationärer Katalysatorschüttung realisierbar. Jedoch ergeben sich in solchen Reaktoren häufig Probleme mit der Thermostatisierung der Katalysatorbetten. Im Allgemeinen werden thermostatisierte Rohrbündelreaktoren verwendet ("isotherme Fahrweise"), welche, besonders bei großen Reaktoren, einen sehr aufwändigen Kühlkreislauf besitzen **(**DE-OS 2 201 528**).** Derartige Reaktoren sind komplex und verursachen hohe Investitionskosten, da die Fertigung eines aus vielen tausenden von Einzelrohren bestehenden Reaktors sehr aufwändig ist und nur von Spezialfirmen zu hohen Kosten durchgeführt werden kann. Hinzu kommen noch mit der Baugröße rasch ansteigende Probleme bezüglich mechanischer Festigkeit und gleichmäßiger Thermostatisierung der Katalysatorschüttung, die den Bau großer Aggregate solchen Typs auch unter rein technischen Gesichtspunkten unpraktikabel machen. Moderne Anlagen im Weltmaßstab, die Produktionskapazitäten von mehreren 100.000 Jahrestonnen haben müssen, nach diesem Prinzip zu bauen ist daher völlig unrealistisch.

Einfache Reaktoren, wie sie für das weiter unten beschriebene erfindungsgemäße Verfahren verwendet werden, enthalten dagegen nur Katalysatorschüttungen auf bzw. zwischen einfachen Auflagerosten und/oder Metallsieben und besitzen kein System zur Wärmehaushaltung im Reaktor, d. h., dass aufwändige Maßnahmen zur Thermostatisierung der Katalysatorbetten, etwa durch Wärmeträgeröl, völlig entfallen. Die Reaktionsenthalpie spiegelt sich bei diesem Reaktortyp in der Temperaturdifferenz zwischen Edukt- und Produktgasstrom quantitativ wieder ("adiabate Fahrweise"). Reaktoren solchen Typs sind leicht vom Technikumsmaßstab (Miniplant-Anlage) in den Maßstab einer großen Produktionsanlage zu übertragen, was die Verfahrensentwicklung erheblich vereinfacht. Letztere umfasst beispielsweise die Feinabstimmung der Fahrparameter wie Druck, Temperatur, Strömungsgeschwindigkeit der Reaktionsgase, Konzentrationen der Reaktionspartner etc., sowie andere Faktoren wie beispielsweise Wahl des Katalysators. Alle diese Faktoren müssen optimal eingestellt sein, um größtmögliche Ausbeute und Selektivität zu erreichen. Die Möglichkeit, diese vielen Variablen in einer relativ kleinen Technikumsanlage optimieren und die Ergebnisse problemlos auf eine große Produktionsanlage übertragen zu können, bringt erhebliche Vorteile, da beispielsweise eine Pilotierung im Prinzip unterbleiben und gleich mit dem Bau einer Anlage mit der erforderlichen Produktionskapazität begonnen werden kann. Darüber hinaus sind Reaktoren solchen Typs in allen Größen preiswert und robust.

GB 1.452.466 befasst sich mit einem Verfahren zur Nitrobenzolhydrierung, bei dem ein adiabater Reaktor einem isothermen Reaktor nachgeschaltet ist. Dabei wird der größte Teil des Nitrobenzols in einem thermostatisierten Rohrbündelreaktor umgesetzt, lediglich die Hydrierung des Restgehaltes an Nitrobenzol erfolgt bei relativ geringem Wasserstoffüberschuss (kleiner 30 : 1) in einem adiabaten Reaktor. Der völlige Verzicht auf einen thermostatisierten Reaktor bei rein adiabater Umsetzung und die damit verbundenen Vorteile werden in GB 1.452.466 jedoch nicht gelehrt.

DE-AS 1 809 711 befasst sich mit dem gleichmäßigen Einbringen von flüssigen Nitroverbindungen in einen heißen Gasstrom durch Verdüsen, bevorzugt an verengten Stellen unmittelbar vor dem Reaktor. Auf den Aufbau des Reaktors wird in DE-AS 1 809 711 nicht eingegangen. Dem Beispiel kann man jedoch entnehmen, dass trotz eines beachtlichen Wasserstoffüberschusses mindestens 34 % der Reaktionsenthalpie den Reaktor nicht mit dem Produktgas verlassen hat, der Reaktor also nicht adiabat betrieben wird.

In DE-OS 3 636 984 wird ein Verfahren zur gekoppelten Produktion von Nitro- und Dinitroaromaten aus den entsprechenden Kohlenwasserstoffen durch Nitrierung und deren nachfolgende Hydrierung beschrieben. Die Hydrierung erfolgt in der Gasphase bei Temperaturen von zwischen 176 und 343,5°C. Es wird eine Apparatur zur Gasphasenhydrierung beschrieben, die im Wesentlichen aus zwei hintereinander geschalteten Reaktoren mit Zwischenkühlung und Eduktzwischeneinspeisung besteht, auf deren Größe und Aufbau nicht eingegangen wird. Jedoch kann man dem Temperaturprofil der Reaktoren entnehmen, dass ein nicht unerheblicher Anteil der Reaktionswärme nicht mit dem Produktgasstrom den Reaktor verlässt. So besitzt der Reaktor Nr. 1 eine Eingangstemperatur von 181,7°C, eine heißeste Stelle von 315,6°C und eine Ausgangstemperatur von 277,2°C; der Reaktor Nr. 2 besitzt eine Eingangstemperatur von 203,9°C, eine heißeste Stelle von 300°C und eine Ausgangstemperatur von 296,7°C. Ob bei einer Umsetzung in technische Größenordnungen von z. B. 80.000 Jahrestonnen die Reaktoren eine Kühleinrichtung benötigen oder nicht, wird in DE-OS 36 36 984 nicht beschrieben. Sowohl in DE-OS 36 36 984 als auch in DE-OS 18 09 711 wird nicht explizit auf die Problematik der Wärmeabfuhr bei Gasphasenhydrierungen eingegangen.

In allen oben genannten Veröffentlichungen werden Kupfer-Katalysatoren eingesetzt, die ausschließlich mit niedrigen Belastungen (< 0,1 g_{Nitroaromat}/[ml_{Katalysator} h]) und auf niedrigem Temperaturniveau betrieben werden. Daraus resultieren geringe Raum-Zeit-Ausbeuten.

Neben den erwähnten Kupfer-Katalysatoren sind zahlreiche andere Kontakte für die Gasphasenhydrierung von Nitroaromaten beschrieben. Sie sind in vielen Publikationen beschrieben und umfassen als hydrieraktive Elemente Pd, Pt, Ru, Fe, Co, Ni, Mn, Re, Cr, Mo, V, Pb, Ti, Sn, Dy, Zn, Cd, Ba, Cu, Ag, Au, und deren Verbindungen, zum Teil als Oxide, Sulfide oder Selenide und auch in Form einer Raney-Legierung sowie auf Trägem, wie Al₂O₃, Fe₂O₃/Al₂O₃, SiO₂, Silikaten, Kohle, TiO₂, Cr₂O₃. Auch diese Katalysatoren werden mit nur geringen Belastungen in einem Temperaturbereich unterhalb 350°C betrieben.

So werden in DE-A 2 244 401 und DE-A 2 849 002 Palladium-Katalysatoren auf Aluminiumoxid-Trägern beschrieben, die als stationäre Katalysatorschüttungen in Wärmetauscherrohren unter Normaldruck bei Belastungen von weniger als 1 g_{Nitroaromat}/[ml_{Katalysator} · h] mit geringen Wasserstoff-Nitrobenzol-Verhältnissen betrieben werden.

In DE-A 4 039 026 werden Palladium-Katalysatoren auf graphitischen Trägern beschrieben, die unter ähnlichen Bedingungen wie die Palladium-Katalysatoren auf Aluminiumoxid betrieben werden. Bei all diesen Verfahrensvarianten muss die große anfallende Reaktionswärme einem technischen Reaktor über ein aufwändiges Wärmeträgersystem entzogen werden.

Mit einem unter rein adiabaten Bedingungen durchgeführten Verfahren befassen sich lediglich die Patente EP 0 696 573 B1**,** EP 0 696 574 B1**,** EP 0 748 789 B1 sowie EP 0 748 790 B1**.** EP 0696574 B1 beschreibt den Prozess zur Herstellung von aromatischen Aminen, in welchem der Katalysator unter adiabaten Bedingungen mit einem Gasgemisch bestehend aus Nitroaromaten und Wasserstoff angeströmt wird, in ganz allgemeiner Weise. In den Verfahren gemäß der Patente EP 0 696 573 B1, EP 0 748 789 B1 sowie EP 0 748 790 B1 werden durch Änderung verschiedener Parameter jeweils bestimmte Vorteile erzielt:

EP 0 696 573 B1 beschreibt den Vorteil besonders hoher Selektivitäten, wenn der umzusetzende Nitroaromat außer mit Wasserstoff auch mit einem Vielfachen des bei der Reaktion entstehenden aromatischen Amins und einem Vielfachen an Wasser über den Katalysator geleitet wird. Bei dieser Fahrweise sind in jedem Katalysatorvolumen pro Mol Nitrogruppe mindestens 2 Mol Aminogruppen und 4 Mol Wasser vorhanden. Die beschriebenen Katalysatoren sind die gleichen wie in EP 0 696 574 B1. Nachteilig bei dieser Verfahrensweise ist, dass große Mengen für die eigentliche Reaktion im Prinzip entbehrlicher Verbindungen, nämlich Wasser und Amin, ständig im Kreis geführt werden müssen. Insbesondere die ständige Rezyklisierung von mindestens 2 Äquivalenten des entstehenden Amins, also des wertvollen Produktes des Verfahrens, ist von großem Nachteil, da das hergestellte Amin dadurch mehrfach thermisch stark belastet wird.

Die Patente EP 0 748 789 B1 und EP 0 748 790 B1 beschreiben die Erzielung von Vorteilen lediglich durch Verwendung spezieller Katalysatorsysteme:
- Palladium-Katalysatoren auf Graphit oder graphithaltigem Koks mit einem Palladiumgehalt > 1,5 und < 7 Massen-% **(**EP 0 748 789 B1**),** mit dem Vorteil außerordentlich langer Standzeiten im Vergleich zu allen früher beschriebenen Katalysatoren. Nachteil dieses Verfahrens sind die mit der hohen Palladiumkonzentration unweigerlich verbundenen immens hohen Kosten des Katalysators. Das Patent geht nicht darauf ein, ob die hohen Katalysatorkosten bei den für eine großtechnische Anwendung erforderlichen großen Mengen an Palladium noch durch die langen Standzeiten kompensiert werden können.
- Palladium-Blei-Katalysatoren auf Graphit oder graphithaltigem Koks mit einem Palladiumgehalt von 0,001 bis 7 Massen-% **(**EP 0 748 790 B1**),** mit dem Vorteil höherer Selektivitäten im Vergleich zu analogen Katalysatoren ohne Bleizusatz. Bei allen im Patent beschriebenen Beispielen kamen Katalysatoren mit 2 Massen-% Palladium zum Einsatz, so dass der Nachteil hoher Katalysatorkosten auch in diesem Fall voll zum Tragen kommt.

Die Durchführung der Hydrierung in Gegenwart von Wasser wird in EP 0 748 789 B1 und EP 0 748 790 B1 jedoch nicht gelehrt.

Die Aufgabe der vorliegenden Erfindung war daher, ein Verfahren zur Herstellung von aromatischen Aminen zur Verfügung zu stellen, das die besonderen Vorteile der oben genannten Verfahren EP 0 696 573 B1, EP 0 748 789 B1 und EP 0 748 790 B1 (lange Standzeiten, hohe Selektivitäten) beinhaltet, ohne deren Nachteile (ständiges Rezyklisieren großer Mengen wertvollen Amins, große Menge einzuspeisenden Wassers, hohe Katalysatorkosten) aufzuweisen. Es wurde nun überraschenderweise gefunden, dass dies gelingt, wenn der umzusetzende Nitroaromat mit Wasserstoff, Wasser, ggf. Stickstoff sowie im Wesentlichen in Abwesenheit von dem aus dem Nitroaromaten hergestellten aromatischen Amin unter Druck bei erhöhter Temperatur über den Katalysator geleitet wird. Diese Vorgehensweise führt auf einfache Weise zu langen Standzeiten und hohen Selektivitäten bei entscheidend verbesserter Wirtschaftlichkeit, da keine oder ggf. nur minimale Mengen des hergestellten aromatischen Amins im Kreis geführt werden. Zudem sind in bevorzugten Ausführungsformen zum einen keine großen Mengen an Wasser einzuspeisen und zum anderen können kostengünstig zugängliche Katalysatoren mit geringeren als den bisher üblichen Edelmetallgehalten eingesetzt werden.

Die Erfindung betrifft ein Verfahren zur Herstellung von aromatischen Aminen der Formel 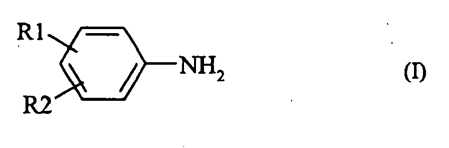 in der R¹ und R² unabhängig voneinander Wasserstoff, Methyl oder Ethyl bedeuten, wobei R¹ zusätzlich Amino bedeuten kann, durch Hydrierung von Nitroaromaten der Formel 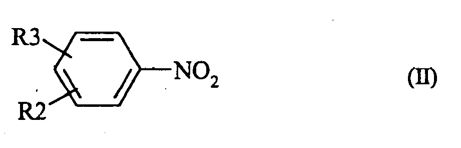 in der R² und R³ unabhängig voneinander Wasserstoff, Methyl oder Ethyl bedeuten, wobei R³ zusätzlich Nitro bedeuten kann,
in der Gasphase mit Wasserstoff an ortsfesten Katalysatoren,
- bei dem die Hydrierung bei einem absoluten Druck von 1 bis 50 bar, bevorzugt 2 bis 20 bar, besonders bevorzugt 2 bis 10 bar und bei einer Eingangstemperatur des eingesetzten Gasgemisches von 150 bis 400°C, bevorzugt 200 bis 300°C, besonders bevorzugt 220 bis 280°C und einer maximalen Katalysatortemperatur von 600°C, bevorzugt 550°C, besonders bevorzugt 500°C unter adiabaten Bedingungen durchgeführt wird, und
- bei dem die ortsfesten Katalysatoren zu Beginn der Hydrierung mit einem Gasgemisch angeströmt werden, das pro Mol Nitrogruppe 3 bis 150 Mol, bevorzugt 6 bis 125 Mol, besonders bevorzugt 12 bis 100 Mol, ganz besonders bevorzugt 50 bis 90 Mol Wasserstoff, sowie 1 bis 100 Mol, bevorzugt 3 bis 50 Mol, besonders bevorzugt 4 bis 25 Mol Wasser enthält, und
- bei dem aus dem bei der Hydrierung erhaltenen Reaktionsgemisch Wasserstoff abgetrennt wird und der im Wesentlichen von dem aromatischen Amin freie Wasserstoff in die Hydrierung zurückgeführt wird, wobei der Anteil des mit dem Wasserstoffstrom in die Hydrierung zurückgeführten aromatischen Amins weniger als 15% des aromatischen Amins, das aus der Hydrierung erhalten worden ist, beträgt.

Bevorzugt wird das Gasgemisch vor Beginn der Hydrierung an den ortsfesten Katalysatoren homogenisiert, d. h. beispielsweise in einem statischen Mischer vermischt.

Bevorzugt sind die ortsfesten Katalysatoren in Form von Katalysatorschüttungen, beispielsweise als Katalysatorfestbett, in dem Reaktor angeordnet. Möglich sind ebenfalls monolithische Reaktoren, deren Wände mit katalytisch aktiven Metallen beschichtet sind.

Die ortsfesten Katalysatoren können dabei in einem Reaktor oder in mehreren in Reihe geschalteten Reaktoren angeordnet sein. Auch ein paralleler Betrieb mehrerer Reaktoren ist möglich. Bei mehreren in Reihe angeordneten Reaktoren bzw. Katalysatorschüttungen bzw. Monolithen wird bevorzugt nur der erste der Reaktoren mit dem frischen Gasgemisch angeströmt. Der nächste Reaktor wird dann bereits mit dem aus dem ersten Reaktor erhaltenen, ggf. beispielsweise mit frischem Wasserstoff und frischem Nitroaromaten aufbereiteten Gasgemisch angeströmt. Es ist jedoch auch die Ausschleusung einzelner Komponenten oder die Zufuhr anderer bzw. weiterer Komponenten zwischen den Reaktoren möglich.

Das erfindungsgemäße Verfahren wird kontinuierlich betrieben. Zu Beginn der Hydrierung (d.h. am Ort des Eintritts des Gasgemisches in den Hydrierreaktor im kontinuierlichen Betrieb) werden die ortsfesten Katalysatoren mit einem Gasgemisch angeströmt, das pro Mol Nitrogruppe 3 bis 150 Mol Wasserstoff sowie 1 bis 100 Mol Wasser enthält. Das bedeutet, dass im kontinuierlichen Betrieb der Reaktor, in dem die ortsfesten Katalysatoren angeordnet sind, mit diesem Gasgemisch angeströmt wird. Wird die Hydrierung in mehreren in Reihe geschalteten Reaktoren durchgeführt, so wird der in Strömungsrichtung erste der Reaktoren im kontinuierlichen Betrieb mit diesem Gasgemisch angeströmt.

Wird im Fall von mehreren in Reihe geschalteten Reaktoren nicht nur nach dem letzten, sondern nach jedem Reaktor das aromatische Amin durch Kondensation abgetrennt, so wird bevorzugt jeder der Reaktoren mit diesem Gasgemisch angeströmt.

In einer bevorzugten Ausführungsform werden bis zu 50 % des im Gasgemisch vor Beginn der Hydrierung enthaltenen Wasserstoffs durch ein Inertgas, bevorzugt Stickstoff, ersetzt. Dann sind in dem Gasgemisch pro Mol Nitrogruppe 3 bis 150 Mol, bevorzugt 6 bis 125 Mol, besonders bevorzugt 12 bis 100 Mol, ganz besonders bevorzugt 50 bis 90 Mol Wasserstoff, 0 bis zu 75 Mol, bevorzugt 0,1 bis zu 62,5 Mol, besonders bevorzugt 0,5 bis zu 50 Mol und ganz besonders bevorzugt 2 bis zu 45 Mol an Inertgas, sowie 1 bis 100 Mol, bevorzugt 3 bis 50 Mol, besonders bevorzugt 4 bis 25 Mol Wasser enthalten. Dies führt zu einer weiteren Erhöhung der ohnehin schon hohen Selektivitäten, mit der bei dem erfindungsgemäßen Verfahren die aromatischen Amine gebildet werden. Die hohen Selektivitäten ermöglichen eine im Vergleich zur eingangs beschriebenen isothermen Fahrweise weniger aufwändige Aufarbeitung der gebildeten aromatischen Amine infolge geringerer Nebenproduktanteile.

Aus dem den Reaktor bzw. den in Strömungsrichtung letzten von mehreren in Reihe bzw. jeden von mehreren parallel geschalteten Reaktoren verlassenden Reaktionsgemisch wird der Wasserstoff bevorzugt dadurch abgetrennt, dass die kondensierbaren Bestandteile des Reaktionsgemisches bevorzugt durch Kondensation zumindest teilweise abgetrennt werden. Dann werden Wasserstoff und ggf. auch Inertgas (Stickstoff) sowie ggf. Wasserdampf in den Reaktor bzw. in den ersten einer Reihe von Reaktoren bzw. jeden von mehreren parallel geschalteten Reaktoren zurückgeführt.

Bevorzugte Nitroaromaten für das erfindungsgemäße Verfahren sind solche der Formel 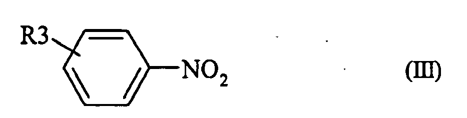 in denen R³ die obige Bedeutung hat.

Besonders bevorzugt als Nitroaromat ist Nitrobenzol. Nitrobenzol kann auf verschiedene Arten hergestellt werden; bevorzugt geschieht dies durch Mononitrierung von Benzol, besonders bevorzugt unter adiabaten Bedingungen wie in EP-A-708 076 beschrieben. EP-A-708 076 offenbart ein Verfahren zur adiabaten Dinitrierung von aromatischen Verbindungen wie beispielsweise Benzol. Dabei offenbart EP-A-708 076 die typischen Reaktionsbedingungen wie z. B. die Temperatur im Reaktorzulauf, die Bedingungen der Dispergierung (einzubringende Dispergierenergie für die Erst-und die Re-Dispergierung und die dafür geeigneten Dispergierorgane) oder die Verweilzeit zwischen den Dispergierschritten sowohl für den ersten Nitrierschritt (zum Nitrobenzol) als auch für den daran anschließenden zweiten Nitrierschritt (zum Dinitrobenzol). Abgesehen von dem für die Dinitrierung benötigten stöchiometrischen Überschuss an Nitriersäure bzw. Salpetersäure zu der aromatischen Verbindung ist die Lehre aus EP-A-708 076 somit auch auf die Mononitrierung von Benzol anwendbar.

Eine für die Durchführung des erfindungsgemäßen Verfahrens geeignete Produktionsanlage enthält bevorzugt mindestens einen adiabaten Reaktor mit stationärem Katalysator. Dabei werden bevorzugt maximal 10, besonders bevorzugt maximal 5, ganz besonders bevorzugt maximal 3 solcher Reaktoren hintereinander angeordnet. Jeder der in Reihe geschalteten Reaktoren kann durch mehrere parallel geschaltete ersetzt werden. Dabei werden bevorzugt maximal 5, besonders bevorzugt maximal 3, ganz besonders maximal 2 Reaktoren parallel geschaltet. Das erfindungsgemäße Verfahren umfasst demnach bevorzugt maximal 50 und minimal einen Reaktor.

Mehrere Reaktoren mit einer Katalysatorschüttung können auch durch eine geringere Anzahl an Reaktoren mit mehreren Katalysatorschüttungen ersetzt werden.

Bevorzugt bestehen die Reaktoren aus einfachen Behältern mit isolierten Katalysatorschüttungen, wie sie z. B. in Ullmanns Encyclopedia of Industrial Chemistry (Fifth, Completely Revised Edition, Vol. B4, Seite 95 -102 und Seite 210 - 216) beschrieben werden.

Die Katalysatorschüttungen werden nach dem Stand der Technik bevorzugt auf oder zwischen gasdurchlässigen Wandungen angebracht. Hierzu werden bevorzugt metallische Auflageroste und/oder Metallsiebe benutzt.

Insbesondere bei dünnen Schüttungen können zusätzlich oberhalb, unterhalb oder oberhalb und unterhalb der Schüttung technische Vorrichtungen zur gleichmäßigen Gasverteilung angebracht werden. Dies können beispielsweise Lochplatten, Glockenböden, Ventilböden oder andere Einbauten sein, die durch Erzeugung eines geringen, aber gleichmäßigen Druckverlusts einen gleichförmigen Eintritt des Gases in die Katalysatorschüttung bewirken.

Die Dicke der Katalysatorschüttungen kann zwischen 1 cm und 5 m, bevorzugt zwischen 5 cm und 2 m, besonders bevorzugt zwischen 10 cm und 1 m, ganz besonders bevorzugt zwischen 30 cm und 60 cm liegen.

Als Katalysatoren können alle bisher für die Gasphasenhydrierung von Nitroverbindungen beschriebenen Kontakte eingesetzt werden. Diese enthalten die weiter oben genannten Elemente, entweder als Legierung oder als Mischoxide und gegebenenfalls auf inertem Trägermaterial. Als Trägermaterialien kommen besonders in Frage: α- und γ-Al₂O₃, SiO₂, TiO₂, Roterde und Limonit, Fe₂O₃/Al₂O₃-Mischungen, CuO/Cr₂O₃-Mischungen, Wasserglas, Graphite, Kokse, Carbon Nanotubes (Kohlenstoffnanoröhren) und Kohlefasern. Es können aber prinzipiell auch andere Träger eingesetzt werden.

Bevorzugt werden in dem erfindungsgemäßen Verfahren die in DE-OS 2 849 002 beschriebenen Katalysatoren eingesetzt. Dies sind Trägerkatalysatoren auf inerten Trägern mit einer BET-Oberfläche von weniger als 20 m²/g, bzw. α-Al₂O₃ mit einer BET-Oberfläche von weniger als 10 m²/g. Die in DE-OS 2 849 002 beschriebene Vorbehandlung mit einer Base ist jedoch nicht unbedingt erforderlich.

Auf dem Trägermaterial sind drei Aktivstoffklassen niedergeschlagen:
(a) 1 - 100 g/l_{Katalysator} eines oder mehrerer Metalle der Gruppen 8 bis 12 des Periodensystems der Elemente (die Bezeichnung der Gruppen des Periodensystems erfolgt hier und im Folgenden nach der IUPAC-Empfehlung von 1986),
(b) 1 - 100 g/l_{Katalysator} eines oder mehrerer Übergangsmetalle der Gruppen 4 bis 6 und 12 sowie
(c) 1 - 100 g/l_{Katalysator} eines oder mehrerer Hauptgruppenelemente der Gruppen 14 und 15.

Elemente der Gruppe 12 können somit als Aktivstoffe (a) und (b) wirken. Bevorzugte Aktivstoffe sind Pd als Metall (a), Ti, V, Nb, Ta, Cr, Mo, W als Übergangsmetall (b) und Pb und Bi als Hauptgruppenelemente (c).

Besonders bevorzugt werden
(a) 5 - 40 g/l_{Katalysator} Pd,
(b) 1 - 40 g/l_{Katalysator} V, ganz besonders bevorzugt 5 - 40 g/l Katalysator und
(c) 2 - 20 g/l_{Katalysator} Pb auf den Träger gebracht.

Die Aktivstoffe werden bevorzugt in Form ihrer löslichen Salze auf den Träger gebracht, gegebenenfalls sind mehrere Behandlungen (Tränkungen) pro Komponente erforderlich.

Die in dem erfindungsgemäßen Verfahren eingesetzten Kontakte werden bevorzugt in einem Temperaturbereich betrieben, der zwischen der Eingangstemperatur des Eduktgases und maximal 600°C, bevorzugt maximal 550°C, besonders bevorzugt maximal 500°C liegt.

Weitere für den Einsatz in dem erfindungsgemäßen Verfahren bevorzugte Katalysatoren sind solche, die Pd alleine oder mit Rh und/oder Ir und/oder Ru auf Kohleträgern mit geringer BET-Oberfläche tragen. Solche Trägermaterialien sind graphithaltig; oder haben graphitähnliche Strukturen, es sind Graphite selbst und Kokse, wie Nadelkoks oder Petrolkoks, sowie Carbon Nanotubes. Diese Träger haben bevorzugt eine BET-Oberfläche von 0,2 - 10 m²/g. Verwendung finden bevorzugt Katalysatoren, die auf Graphit oder graphithaltigem Koks oder Carbon Nanotubes als Träger 0,001 - 1,5 Massen-% Pd, bezogen auf die Gesamtmasse des Katalysators, enthalten, wobei bis zu 40 % des eingesetzten Palladiums durch Ir und/oder Rh und/oder Ru ersetzt werden kann. Diese Katalysatoren enthalten also das (die) Edelmetall(e) in folgenden Anordnungen auf dem Träger: Pd alleine, Pd/Ir, Pd/Rh, Pd/Ru, Pd/Ir/Rh, Pd/Ir/Ru, Pd/Rh/Ru, Pd/Ir/Rh/Ru. In vielen Fällen wird eine der genannten Zweierkombinationen oder Pd alleine eingesetzt. In bevorzugter Weise liegt in den Katalysatoren auf Kohleträgern das Palladium in einer Menge von 0,005 - 1 Massen-%, bevorzugt 0,05 - 0,5 Massen-%, bezogen auf das Gesamtmasse des Katalyators, vor. Falls neben Palladium andere Metalle der sog. "Platingruppe" (Ru, Rh, Pd, Os, Ir, Pt) eingesetzt werden, so beträgt deren Anteil bevorzugt in der Summe 10 - 40 % bezogen auf die Masse des eingesetzten Palladiums; untereinander beträgt ihr Massenverhältnis bevorzugt 1 : 1 - 3 : 1 zwischen je zweien.

Es hat sich weiterhin als vorteilhaft erwiesen, die genannten Katalysatoren zusätzlich mit einer schwefelhaltigen oder phosphorhaltigen, bevorzugt phosphorhaltigen Verbindung zu dotieren. Ein solcher zusätzlicher Gehalt an Dotierungsmittel beträgt bevorzugt 0,1 - 2 Massen-%, bevorzugt 0,1 - 1 Massen-% Schwefel oder Phosphor, bevorzugt Phosphor, in chemisch gebundener Form, bezogen auf die Gesamtmasse des Katalysators. Als phosphorhaltige Verbindungen für die Dotierung der erfindungsgemäßen Katalysatoren sind bevorzugt zu nennen: die Sauerstoffsäuren des Phosphors H₃PO₄, H₃PO₃, H₃PO₂ oder deren Alkalisalze, wie z. B. Natriumdihydrogenphosphat, Natrium- oder Kaliumphosphat oder Natriumhypophosphit.

Zur Herstellung der Katalysatoren auf Kohleträgern kann so vorgegangen werden, dass auf einen der genannten Träger in Form von Pillen, Kugeln, Stranggranulat oder Bruchstücken von etwa 0,5 - 10 mm Abmessung die genannten Edelmetalle (Pd alleine oder mit Rh und/oder Ir und/oder Ru) in Form geeigneter Salze sowie die schwefelhaltige oder phosphorhaltige Verbindung in getrennten Arbeitsgängen aufgetragen werden, wobei nach jedem Auftrag getrocknet wird. Das Trocknen geschieht in bekannter Weise, beispielsweise bei 100 - 140°C und vermindertem bis normalem Druck, beispielsweise bei 1 - 1.000 mbar; als verminderter Druck kommt beispielsweise der einer Wasserstrahlpumpe in Betracht. Zum Tränken des Trägers können wässrige Lösungen verwendet werden. Dies ist in bevorzugter Weise bei den schwefel- oder phosphorhaltigen Verbindungen, von denen wasserlösliche bevorzugt werden, der Fall. Die Edelmetallsalze können auch in organischen Lösungsmitteln, wie einfachen Alkoholen, Ketonen, cyclischen Ethern oder Nitrilen, gelöst und aufgetragen werden. Beispiele für solche organischen Lösungsmittel sind Methanol, Ethanol, Propanol, Isopropanol, Aceton, Methyl-Ethylketon, Dioxan, Acetonitril und vergleichbare Lösungsmittel. Bei Salzen mit organischen Anionen können auch Methylenchlorid und vergleichbare Lösungsmittel eingesetzt werden. Geeignete Salze der Edelmetalle sind beispielsweise ihre Chloride, Nitrate oder Acetate.

Nach dem Imprägnieren und dem abschließenden Trocknen kann ein solcher Katalysator auf Kohleträgern in dem erfindungsgemäßen Verfahren eingesetzt werden. Er kann aber auch vor Beginn der Hydrierung des Nitroaromaten durch eine Behandlung mit Wasserstoff bei erhöhter Temperatur aktiviert werden. Eine solche erhöhte Temperatur liegt beispielsweise im Bereich von 200 - 400°C, bevorzugt im Bereich von 200 - 380°C.

Die Katalysatoren auf Kohleträgern werden ebenfalls in einem Temperaturbereich bis maximal 600°C, bevorzugt maximal 550°C, besonders bevorzugt maximal 500°C betrieben.

Alle beschriebenen Katalysatoren, unabhängig von Trägermaterial und Art der Aktivmetalle, können bei Aktivitätsabfall leicht *in situ,* d. h. im Hydrierreaktor selbst, mit Luft oder sauerstoffhaltigen Gasgemischen regeneriert werden. Im Fall der Katalysatoren auf Kohleträgern ist eine solche Regenerierung nicht möglich bei Verwendung von anderen als den beschriebenen Kohleträgern, z.B. bei aktivierter Kohle als Träger, da eine aktivierte Kohle bei einer solchen Regenerierung zu verbrennen beginnt. Zur erneuten Aktivierung des Katalysators kann sich eine Behandlung mit Wasserstoff bei 200 - 400°C anschließen.

Die als bevorzugt beschriebenen Kontakte ermöglichen eine besonders lange Laufzeit in dem erfindungsgemäßen Verfahren.

Prinzipiell können die Katalysatorkörner jede beliebige Form aufweisen, wie z.B. Kugeln, Stäbchen, Raschigringe, Pallringe oder Granulat oder Tabletten. Die mittlere Partikelgröße liegt typischerweise zwischen 100 und 5.000µm. Bevorzugt werden Formkörper benutzt, deren Schüttungen einen niedrigen Strömungswiderstand bei gutem Gas-Oberflächen-Kontakt aufweisen, wie z.B. Raschigringe, Sattelkörper, Wagenräder und Spiralen.

Statt Katalysatorschüttungen können auch als Trägermaterial geeignete Packungen verwendet werden. Dies wären z.B. Wabenkörper, Monolithe oder gewellte Schichten. Diese Packungen werden zum erfindungsgemäßen Einsatz natürlich vor dem Einbringen in den Reaktor durch das Aufbringen geeigneter Metallverbindungen aktiv gemacht.

Die Belastung der Katalysatoren in dem erfindungsgemäßen Verfahren kann sehr hoch sein und 0,1 bis zu 20, bevorzugt bis zu 15, besonders bevorzugt bis zu 10 und ganz besonders bevorzugt bis zu 5 g_{Nitroaromat}/[ml_{Katalysator} · h] betragen. Im Falle mehrerer in Reihe geschalteter Reaktoren kann die Belastung von Reaktor zu Reaktor variiert werden. Bevorzugt liegt die Belastung jedoch in allen Reaktoren in dem Bereich von 0,1 bis zu 20 g_{Nitroaromat}/[ml_{Katalysator} · h]. Das erfindungsgemäße Verfahren zeichnet sich demnach durch hohe Raum-Zeit-Ausbeuten aus.

Die ortsfesten Katalysatoren können in einem Reaktor oder in mehreren in Reihe geschalteten oder parallel betriebenen Reaktoren angeordnet sein. Im Falle von mehreren in Reihe geschalteten Katalysatorschüttungen werden, nachdem der Gasstrom eine Katalysatorschüttung durchlaufen hat, vor der nun folgenden Katalysatorschüttung bevorzugt frischer Nitroaromat und Wasserstoff in den Gasstrom eindosiert, um die verbrauchten Anteile zu ersetzen und um ggf. eine andere Belastung mit Nitroaromaten einzustellen.

Die Verdampfung des Nitroaromaten kann so erfolgen, wie in DE 1 809 711 beschrieben, bevorzugt wird jedoch der Nitroaromat im Frischwasserstoff vollständig verdampft und dann gasförmig in den Kreisgasstrom gegeben. Der Vorteil dieser Verfahrensweise liegt in der deutlich geringeren Bildung von Ablagerungen im Reaktor und in den Zuleitungen. Die Verdampfung kann nach dem Stand der Technik in bekannten Verdampfern erfolgen, wie z. B. Fallfilm-, Steigrohr-, Einspritz-, Dünnschicht-, Umlauf- und Wendelrohrverdampfern.

Weiterhin ist die Verdüsung des flüssigen Nitroaromaten in den Frischwasserstoff- oder Kreisgaswasserstoffstrom mittels Einstoff- oder Zweistoffdüsen möglich, wobei die Vereinigung des Eduktgasstromes nach einer Überhitzung in einem Wärmetauscher erfolgen kann. Der Verdampfung kann eine grundsätzlich bekannte Tröpfchenabscheidung nachgeschaltet werden. Der Eduktgasstrom wird in bekannter Weise mittels entsprechender Zuführung und Verteilung und/oder durch Vermischungseinrichtungen im Kreisstrom vermischt.

In einer besonderen Ausführungsform des Verfahrens wird eine zusätzliche Schüttung aus inertem Material in Anströmrichtung vor dem heterogenen Katalysator angebracht, wobei der Partikeldurchmesser im Vergleich zum Katalysator selbst um den Faktor 1,5 - 100 größer ist. Dies hat zum Vorteil, dass bei der Verdüsung evtl. nicht verdampfte Tropfen der eingesetzten Nitroverbindung abgeschieden und weiter verdampft werden können, bevor diese mit der Katalysatorschüttung in Kontakt kommen. Die hierzu verwendeten Partikel können wahlweise noch mit einem Oxidationskatalysator, bevorzugt einem Oxid des Vanadiums, imprägniert werden.

Bevorzugt wird nach jeder Katalysatorschüttung das sie verlassende Reaktionsgemisch unter Dampfgewinnung (bevorzugt Wasserdampf) auf die Eingangstemperatur des nächsten Reaktors abgekühlt. Dazu leitet man es bevorzugt durch einen oder mehrere Wärmetauscher. Dies können die dem Fachmann bekannten Wärmetauscher, wie z. B. Rohrbündel-, Platten-, Ringnut-, Spiralstrom-, Rippenrohrwärmetauscher sein.

Nach Verlassen des Reaktors bzw. des in Strömungsrichtung letzten von mehreren in Reihe geschalteten Reaktoren wird das Produktgas zunächst zur Überhitzung und Erzeugung von Dampf (bevorzugt Wasserdampf) verwendet. Bevorzugt nur nach diesem Schritt wird das Gasgemisch soweit abgekühlt, dass aromatisches Amin aus dem Reaktionsgemisch durch Kondensation entfernt werden kann.

In einer besonders wirtschaftlichen Variante des Verfahrens wird das gasförmig anfallende Reaktionswasser bevorzugt nur unvollständig auskondensiert und der verbliebene Wasserdampf zusammen mit dem restlichen Kreisgas rezyklisiert, so dass auf eine externe Zugabe von Wasser verzichtet werden kann.

In dem erfindungsgemäßen Verfahren wird aus dem in der Hydrierung erhaltenen Reaktionsgemisch Wasserstoff abgetrennt und der im Wesentlichen von dem aromatischen Amin freie Wasserstoff in die Hydrierung zurückgeführt. Bevorzugt erfolgt die Abtrennung des Wasserstoffs durch weitgehende Kondensation des aromatischen Amins und zumindest teilweise Kondensation von Wasserdampf. Der so erhaltene, Wasserstoff und bevorzugt auch Wasserdampf enthaltende, Gasstrom wird dann, ggf. nach weiteren Aufarbeitungsschritten, in die Hydrierung zurückgeführt. Bevorzugt wird der zurückgeführte Gasstrom zunächst mit frischem Nitrobenzol und zusätzlichem Wasserstoff angereichert und dann in die Hydrierung geführt.

Bei der Kondensation wird der mit technisch und wirtschaftlich vertretbarem Aufwand kondensierbare Anteil an aromatischem Amin auskondensiert. Der Anteil des mit dem Wasserstoffstrom in die Hydrierung zurückgeführten aromatischen Amins beträgt daher weniger als 15 %, bevorzugt weniger als 10 %, besonders bevorzugt weniger als 5 % des aromatischen Amins, das aus der Hydrierung erhalten worden ist. Daher werden die ortsfesten Katalysatoren in dem Reaktor bzw. in dem in Strömungsrichtung ersten von mehreren in Reihe geschalteten Reaktoren in dem erfindungsgemäßen Verfahren bevorzugt mit einem Gasgemisch angeströmt, das pro Mol Nitrogruppe maximal 0,4 Mol Aminogruppen in Form des hergestellten aromatischen Amins aufweist. Diese ggf. auftretenden geringen Restkonzentrationen an zurückgeführtem Amin resultieren daraus, dass eine vollständige Abscheidung des Produktes unter großtechnisch realisierbaren Bedingungen in der Regel nicht notwendig und auch nicht wirtschaftlich ist.

Der Kreisgasstrom durchläuft daraufhin bevorzugt einen oder mehrere Kompressor(en), um den Strömungswiderstand von Reaktoren und Wärmeaustauschern auszugleichen und den Massenstrom des Kreisgases zu steuern.

Die Kompressoren können einfache, bekannte Maschinen (z. B. Flüssigkeitsringpumpen, Drehkolbengebläse, Turbogebläse oder -verdichter) sein, da der Druckverlust durch die Bauweise der Reaktoren klein gehalten werden kann. Bevorzugt werden trocken laufende Verdichter benutzt.

Vor dem Reaktor bzw. dem in Strömungsrichtung ersten von mehreren in Reihe geschalteten Reaktoren bzw. allen von mehreren parallel geschalteten Reaktoren wird der von aromatischem Amin weitgehend befreite ausgekreiste Wasserstoff wieder in den Kreisgasstrom eingeleitet (rezyklisiert).

Das Kondensat wird in eine technische Einrichtung zur Trennung flüssiger Phasen geleitet, und die wässrige und die organische Phase werden getrennt aufgearbeitet.

Aus der wässrigen Phase gewonnenes aromatisches Amin wird der Aufarbeitung der organischen Phase zugeführt. Die Aufarbeitungen erfolgen in bekannter Weise durch Destillation bzw. durch Strippen mit Wasserdampf.

Bevorzugt wird das Kreisgas unmittelbar vor dem Reaktor mittels eines Wärmetauschers wieder auf die Eingangstemperatur von 150 bis 400 °C gebracht. Davor oder danach, bevorzugt danach, werden Nitroaromat und Frischwasserstoff wie oben beschrieben eindosiert.

Durch die Fahrweise nach dem erfindungsgemäßen Verfahren kommt man bei minimalem Kreisgasvolumen zu besonders langen Standzeiten und außerordentlich hohen Selektivitäten. Ursache für die langen Standzeiten ist das Vorhandensein von Wasser im Reaktionsgas. Dies hat eine Verzögerung der Desaktivierung des Katalysators durch Verkokung zur Folge. Wassermoleküle konkurrieren erfolgreich mit organischen Molekülen um die freien Zentren auf der Katalysatoroberfläche, wodurch die Verweilzeit der organischen Moleküle herabgesetzt und somit der Desaktivierungsprozess verlangsamt wird, wobei die dafür notwendige Wassermenge noch geringer sein kann als in EP 0 696 573 B1 beschrieben.

Der weitgehende Verzicht auf die Rückführung des gebildeten Amins bringt beträchtliche wirtschaftliche Vorteile, da nicht ständig große Mengen des wertvollen Produktes im Kreis geführt werden müssen. Zum Vergleich: in EP 0 696 573 B1 ist die Menge des vor dem ersten ortsfesten Katalysator zugeführten Amins wenigstens fünfmal größer (dort minimal 2 Mol Aminogruppen pro Mol Nitrogruppe, hier maximal 0,4 Mol Aminogruppen pro Mol Nitrogruppe). Im Prinzip kann die Rückführung des Amins auch ganz unterbleiben; sie ist lediglich die Folge der technischen und wirtschaftlichen Rahmenbedingungen in großen Produktionsanlagen, die eine vollständige Abscheidung des gebildeten Amins unpraktikabel machen.

Eine besondere Selektivitätssteigerung, speziell bei Anfahrprozessen, kann auf einfache Weise durch Zugabe von Stickstoff erzielt werden, wie auch den Beispielen entnommen werden kann.

### Beispiele

### Beispiel 1 (Vergleichsbeispiel) - Verzicht auf Wasser im Eduktstrom

In der nachstehend beschriebenen Anlage wird ein Katalysator eingesetzt, der Palladium (18 g/l_{Katalysator}; entspricht etwa 1,8 Massen-% Palladium), Vanadium (18 g/l_{Katalysator}) und Blei (6 g/l_{Katalysator}) auf einem α-Al₂O₃-Träger mit einem mittleren Partikeldurchmesser von 1,6 mm und einer BET-Oberfläche von 5 m²/g enthält.

Als Versuchsanlage dient eine Miniplant-Anlage mit drei hintereinander geschalteten Reaktoren. Die Anlage verfügt über kein geschlossenes Kreisgassystem zur Rezyklisierung des Wasserstoffs. Mittels Dosierpumpen wird Nitrobenzol von oben in die Verdampfer gepumpt. Analog kann mittels einer Dosierpumpe rezyklisiertes Reaktionswasser in den Verdampfer gepumpt werden. Der Wasserstoff wird von unten in die Verdampfer geleitet, die durch Ölthermostaten beheizt werden (ca. 250°C), so dass das von oben eingepumpte Nitrobenzol im Gegenstrom verdampfen kann. Die Wasserstoff-Versorgung wird vor den Verdampfern durch Massedurchflussregler geregelt, der Druck in der Anlage über ein Druckhalteventil nach der Ausspeisung eingestellt.

Die Reaktoren bestehen aus jeweils einem Edelstahlrohr. Auf einem Sieb wird eine Schüttung aus inerten Edelstahl-Wendeln und α-Al₂O₃ platziert. Auf diesen inerten Schichten liegt der Katalysator (Teilchendurchmesser ca. 1,6 mm, 400 mm Schichthöhe), gefolgt von einer weiteren inerten Schüttung. Je Reaktor wird so eine Schüttung von ca. 130 ml Katalysator erhalten. Durch die Schüttungen hindurch ragt jeweils ein Innenrohr, das ein in der Höhe verschiebbares Thermoelement enthält, um den axialen Temperaturverlauf durchgängig bestimmen zu können.

Das Trägergas wird nach Austritt aus Reaktor 1 bzw. Reaktor 2 mit Marlothermöl auf die gewünschte Eintrittstemperatur von Reaktor 2 bzw. Reaktor 3 gekühlt. Eine Kondensation von Reaktionsprodukten findet zwischen diesen Reaktoren nicht statt. Hinter Reaktor 3 wird das Reaktionsprodukt mit Wasser gekühlt, die nicht flüchtigen Bestandteile auskondensiert und in einem nachgeschalteten Abscheider von den gasförmigen Komponenten getrennt.

Die flüssigen Bestandteile werden aus dem Abscheider in den Produktsammelbehälter geführt und dort aufgefangen (Glasbehälter). Aus diesem Behälter kann eine Flüssigkeitsprobe entnommen werden, die einem Durchschnittswert über einen längeren Zeitraum entspricht (Totalkondensat). Die Analyse der Flüssigkeitsproben erfolgt gaschromatographisch.

Bei einer durchschnittlichen Belastung von 1,2 g_{Nitroaromat}/[ml_{Katalysator} · h] (Reaktor 1), bzw. 1,5 g_{Nitroaromat}/[ml_{Katalysator} · h] (Reaktoren 2 und 3) und einem Wasserstoff: Nitrobenzol-Verhältnis von ca. 80 : 1 wird ca. 450 Stunden lang Nitrobenzol-freies Anilin produziert. Danach steigt der Nitrobenzolgehalt im Totalkondensat rasch auf 3500 ppm an, und der Versuch wird abgebrochen.

### Beispiel 2 (erfindungsgemäßes Beispiel)

Es werden die in Beispiel 1 genannten Bedingungen eingehalten und zusätzlich 2 Mol Wasser pro Mol Nitrobenzol in den ersten Reaktor eingespeist. Nach 460 Stunden kann bereits sicher festgestellt werden, dass die Wanderungsgeschwindigkeit der Reaktionszone durch den ersten Reaktor wesentlich langsamer ist als in Beispiel 1; es sind keinerlei Anzeichen eines Nitrobenzoldurchbruchs erkennbar. Der Versuch wird daher aus Gründen der Zeitersparnis abgebrochen. Durch Extrapolation wird ermittelt, dass durch die Zudosierung von Wasser die Laufzeit um mehr als das Doppelte auf ca. 1100 Stunden ansteigt.

### Beispiel 3 (erfindungsgemäßes Beispiel)

Der Versuch wird in einer Miniplant-Anlage durchgeführt, die analog zu der in Beispiel 1 beschriebenen aufgebaut und zusätzlich noch mit einem Kreisgaskompressor zur Rezyklisierung der Gasströme ausgerüstet ist.

In dieser Anlage wird ein Katalysator eingesetzt, der Palladium (9 g/l_{Katalysator}; entspricht ca. 0,9 Massen-% Palladium), Vanadium (9 g/l_{Katatysator}) und Blei (3 g/l_{Katalysator}) auf einem α-Al₂O₃-Träger mit einem mittleren Partikeldurchmesser von 1,6 mm und einer BET-Oberfläche von ca. 5 m²/g enthält. Bei einer durchschnittlichen Gesamtbelastung von ca. 1,5 g_{Nitroaromat}/[g_{Katalysator} · h], einem durchschnittlichen molaren Wasserstoff : Nitrobenzol-Verhältnis von ca. 90 : 1, einem molaren Wasser : Nitrobenzol-Verhältnis vor dem ersten Reaktor von ca. 4 : 1 und einem Druck, der sukzessive von 3 auf 5 bar_{(abs)} gesteigert wird, kann eine Zykluszeit von 1500 Stunden erzielt werden (Abbruchkriterium: die Lage der Reaktionszone ist in allen Reaktoren kurz vor dem Ende der Schüttung (es wird aber noch kein Nitrobenzoldurchbruch im Totalkondensat beobachtet).). Die über den gesamten Zyklus gemittelte Anilinselektivität beträgt 99,7%.

### Beispiel 4 (erfindungsgemäßes Beispiel) - geringe Stickstoffkonzentration

Es werden die in Beispiel 3 genannten Bedingungen eingehalten mit Ausnahme des Drucks (hier konstant 4 bar_{(abs)}) und des prozentualen Molverhältnisses Wasserstoff: Stickstoff. Letzteres wird auf einen Wert von ca. 90 : 10 eingestellt.

### Beispiel 5 (erfindungsgemäßes Beispiel) 4 - hohe Stickstoffkonzentration

Es werden die in Beispiel 4 genannten Bedingungen eingehalten mit Ausnahme des prozentualen Molverhältnisses Wasserstoff: Stickstoff; dieses wird beginnend bei ca. 50 : 50 innerhalb von 75 Stunden sukzessive auf ca. 80 : 20 erhöht.

Die in den Beispielen 4 und 5 beschriebenen Versuche werden aus Zeitgründen frühzeitig abgebrochen, da nur der Einfluss der Stickstoffkonzentration auf die Anfahrselektivität untersucht werden soll. Es wird festgestellt, dass ein teilweiser Ersatz des überstöchiometrisch eingesetzten Wasserstoffs durch Stickstoff die Selektivität in erheblichem Maße positiv beeinflusst. So wird in Beispiel 5 bereits nach 2 Stunden eine Selektivität > 99 % erreicht; in Beispiel 4 ist dies erst nach 30 Stunden, in Beispiel 3 (völlig ohne Stickstoff) sogar erst nach 75 Stunden der Fall.

## Patentansprüche

1. Verfahren zur Herstellung von aromatischen Aminen der Formel 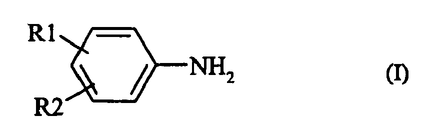 in der R¹ und R² unabhängig voneinander Wasserstoff, Methyl oder Ethyl bedeuten, wobei R¹ zusätzlich Amino bedeuten kann, durch Hydrierung von Nitroaromaten der Formel 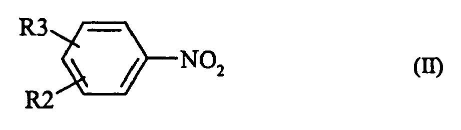 in der R² und R3 unabhängig voneinander Wasserstoff, Methyl oder Ethyl bedeuten, wobei R³ zusätzlich Nitro bedeuten kann,
mit Wasserstoff an ortsfesten Katalysatoren,
• bei dem die Hydrierung bei einem absoluten Druck von 1 bis 50 bar und bei einer Eingangstemperatur des eingesetzten Gasgemisches von 150 bis 400°C und einer maximalen Katalysatortemperatur von 600°C unter adiabaten Bedingungen durchgeführt wird, und
• bei dem die ortsfesten Katalysatoren zu Beginn der Hydrierung mit einem Gasgemisch angeströmt werden, das pro Mol Nitrogruppe 3 bis 150 Mol Wasserstoff sowie 1 bis 100 Mol Wasser enthält, und
• bei dem aus dem bei der Hydrierung erhaltenen Reaktionsgemisch Wasserstoff abgetrennt wird und der im Wesentlichen von dem aromatischen Amin freie Wasserstoff in die Hydrierung zurückgeführt wird, wobei der Anteil des mit dem Wasserstoffstrom in die Hydrierung zurückgeführten aromatischen Amins weniger als 15% des aromatischen Amins, das aus der Hydrierung erhalten worden ist, beträgt.

2. Verfahren nach Anspruch 1, bei dem Nitroaromaten der Formel 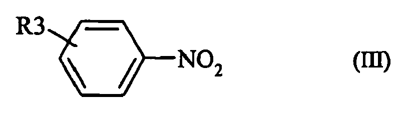 eingesetzt werden in denen R³ Wasserstoff, Methyl, Ethyl oder Nitro bedeutet.

3. Verfahren nach Anspruch 1, bei dem die Hydrierung in 1 -10 hintereinander geschalteten Reaktoren durchgeführt wird, von denen jeder Reaktor durch maximal 5 parallel geschaltete Reaktoren ersetzt werden kann.

4. Verfahren nach Anspruch 1, bei dem das in der Hydrierung erhaltene Reaktionsgemisch abgekühlt wird und die abgeführte Wärme zur Dampferzeugung eingesetzt wird.

5. Verfahren nach Anspruch 3, bei dem dem eingesetzten Gasgemisch vor jedem Reaktor Nitroaromat und Wasserstoff zugeführt werden.

6. Verfahren nach Anspruch 1, bei dem die ortsfesten Katalysatoren in Form von Katalysatorschüttungen in einer durchströmten Dicke von zwischen 1 cm und 5 m vorliegen.

7. Verfahren nach Anspruch 1, bei dem ein Katalysator eingesetzt wird, der
(a) 1 -100 g/l_{Katalysator} eines oder mehrerer Metalle der Gruppen 8 bis 12 des Periodensystems der Elemente, und
(b) 1 - 100 g/l_{Katalysator} eines oder mehrerer Übergangsmetalle der Gruppen 4 bis 6 und 12, sowie
(c) 1 - 100 g/l_{Katalysator} eines oder mehrerer Hauptgruppenelemente der Gruppen 14 und 15, auf einem Träger mit einer BET-Oberfläche von weniger als 20 m²/g enthält.

8. Verfahren nach Anspruch 7, bei dem ein Katalysator eingesetzt wird, der 5 - 40 g Pd, 1 - 40 g Ti, V, Nb, Ta, Cr, Mo und/oder W und 2 - 20 g Pb und/oder Bi pro Liter Katalysator auf α-Al₂O₃ enthält.

9. Verfahren nach Anspruch 1, bei dem ein Palladium auf Kohleträgern enthaltender Katalysator eingesetzt wird, dessen Träger eine BET-Oberfläche von 0,2 - 10 m²/g aufweist, und dessen Palladiumgehalt 0,001 - 1,5 Massen-%, bezogen auf die Gesamtmasse des Katalysators, beträgt.

## Claims

1. Process for preparing aromatic amines of the formula 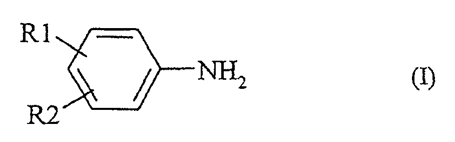 where R¹ and R² are each, independently of one another, hydrogen, methyl or ethyl and R¹ can also be amino, by hydrogenation of nitroaromatics of the formula 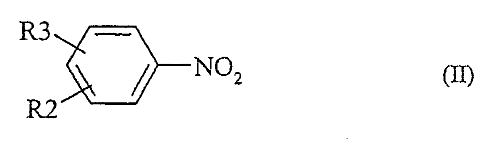 where R² and R³ are each, independently of one another, hydrogen, methyl or ethyl and R³ can also be nitro,
by means of hydrogen over fixed catalysts,
• in which the hydrogenation is carried out at an absolute pressure of from 1 to 50 bar and an inlet temperature of the gas mixture used of from 150 to 400°C and a maximum catalyst temperature of 600°C under adiabatic conditions and
• in which a gas mixture containing from 3 to 150 mol of hydrogen and from 1 to 100 mol of water per mole of nitro group is passed over the fixed catalysts at the beginning of the hydrogenation and
• in which hydrogen is separated off from the reaction mixture obtained in the hydrogenation and the hydrogen which is essentially free of the aromatic amine is recirculated to the hydrogenation, with the proportion of the aromatic amine recirculated together with the hydrogen stream to the hydrogenation being less than 15% of the aromatic amine which has been obtained from the hydrogenation.

2. Process according to Claim 1, wherein nitroaromatics of the formula 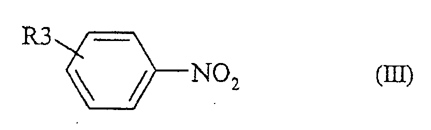 in which R³ is hydrogen, methyl, ethyl or nitro are used.

3. Process according to Claim 1, wherein the hydrogenation is carried out in 1 - 10 reactors connected in series, of which each reactor can be replaced by a maximum of 5 reactors connected in parallel.

4. Process according to Claim 1, wherein the reaction mixture obtained in the hydrogenation is cooled and the heat removed is used for steam generation.

5. Process according to Claim 3, wherein nitroaromatic and hydrogen are introduced into the feed gas mixture upstream of each reactor.

6. Process according to Claim 1, wherein the fixed catalysts are in the form of catalyst beds having a thickness through which flow occurs of from 1 cm to 5 m.

7. Process according to Claim 1, wherein a catalyst containing
(a) 1 - 100 g/l_{catalyst} of one or more metals of groups 8 to 12 of the Periodic Table of the Elements,
(b) 1 - 100 g/l_{catalyst} of one or more transition metals of groups 4 to 6 and 12 and
(c) 1 - 100 g/l_{catalyst} of one or more main group elements of groups 14 and 15
on a support having a BET surface area of less than 20 m²/g is used.

8. Process according to Claim 7, wherein a catalyst containing 5 - 40 g of Pd, 1 - 40 g of Ti, V, Nb, Ta, Cr, Mo and/or W and 2 - 20 g of Pb and/or Bi per litre of catalyst on α-Al₂O₃ is used.

9. Process according to Claim 1, wherein a catalyst containing palladium on carbon supports whose support has a BET surface area of 0.2 - 10 m²/g and whose palladium content is 0.001 - 1.5% by mass, based on the total mass of the catalyst, is used.

## Revendications

1. Procédé pour la production d'amines aromatiques de formule 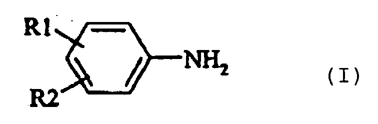 dans laquelle R¹ et R² représentent indépendamment l'un de l'autre un atome d'hydrogène, un groupe méthyle ou éthyle, R¹ pouvant en outre représenter un groupe amino, par hydrogénation de composés aromatiques nitrés de formule 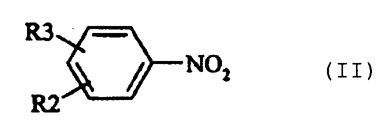 dans laquelle R² et R³ représentent indépendamment l'un de l'autre un atome d'hydrogène, un groupe méthyle ou éthyle, R³ pouvant en outre représenter un groupe nitro,
avec de l'hydrogène sur des catalyseurs fixes,
• dans lequel l'hydrogénation est effectuée dans des conditions adiabatiques sous une pression absolue de 1 à 50 bars et à une température d'entrée du mélange gazeux utilisé de 150 à 400 °C et à une température maximale du catalyseur de 600 °C, et
• dans lequel au début de l'hydrogénation on fait passer sur les catalyseurs fixes un mélange gazeux qui contient par mole de groupe nitro 3 à 150 moles d'hydrogène ainsi que 1 à 100 moles d'eau, et
• dans lequel on sépare l'hydrogène du mélange réactionnel obtenu à partir de l'hydrogénation et on renvoie dans l'hydrogénation l'hydrogène pratiquement exempt de l'amine aromatique, la proportion de l'amine aromatique renvoyée avec le courant d'hydrogène dans l'hydrogénation étant inférieure à 15 % de l'amine aromatique qui a été obtenue à partir de l'hydrogénation.

2. Procédé selon la revendication 1, dans lequel on utilise des composés aromatiques nitrés de formule 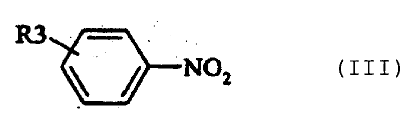 dans lesquels R³ représente un atome d'hydrogène, un groupe méthyle, éthyle ou nitro.

3. Procédé selon la revendication 1, dans lequel on effectue l'hydrogénation dans 1 - 10 réacteurs montés en série, dont chaque réacteur peut être remplacé par au maximum 5 réacteurs montés en parallèle.

4. Procédé selon la revendication 1, dans lequel on refroidit le mélange réactionnel obtenu dans l'hydrogénation et on utilise la chaleur dissipée pour la production de vapeur.

5. Procédé selon la revendication 3, dans lequel avant chaque réacteur on ajoute du composé nitré aromatique et de l'hydrogène au mélange gazeux utilisé.

6. Procédé selon la revendication 1, dans lequel les catalyseurs fixes se trouvent sous forme de lits de catalyseurs en une épaisseur traversée comprise entre 1 cm et 5 m.

7. Procédé selon la revendication 1, dans lequel on utilise un catalyseur qui contient
(a) 1 - 100 g/l_{catalyseur} d'un ou plusieurs métaux des groupes 8 à 12 du système périodique des éléments, et
(b) 1 - 100 g/l_{catalyseur} d'un ou plusieurs métaux de transition des groupes 4 à 6 et 12, ainsi que
(c) 1 - 100 g/l_{catalyseur} d'un ou plusieurs éléments des groupes principaux des groupes 14 et 15,
sur un support ayant une surface BET de moins de 20 m²/g.

8. Procédé selon la revendication 7, dans lequel on utilise un catalyseur qui contient 5 - 40 g de Pd, 1 - 40 g de Ti, V, Nb, Ta, Cr, Mo et/ou W et 2 - 20 g de Pb et/ou Bi par litre de catalyseur sur α-Al₂O₃.

9. Procédé selon la revendication 1, dans lequel on utilise un catalyseur contenant du palladium sur supports en charbon, dont le support présente une surface BET de 0,2 - 10 m²/g, et dont la teneur en palladium vaut 0,001 - 1,5 % en masse, par rapport à la masse totale du catalyseur.
